# EUROPÄISCHE PATENTSCHRIFT

(11) **EP 1 102 749 B1**
(45) Veröffentlichungstag und Bekanntmachung des Hinweises auf die Patenterteilung: **31.10.2007**
(21) Anmeldenummer: 99938237.7
(22) Anmeldetag: 14.07.1999
(51) Int. Cl.: C07D 213/80, C07D 213/78, C07B 43/00

(54) **VERFAHREN ZUR HERSTELLUNG VON 2-HALOGENNIKOTINSÄUREDERIVATEN UND 2-CHLORNIKOTINSÄURE-N-BUTYLESTER ALS ZWISCHENPRODUKT**
METHOD FOR PRODUCING 2-HALOGEN NICOTINIC ACID DERIVATIVES AND 2-CHLORONICOTINIC ACID-N-BUTYL ESTERS AS INTERMEDIATE PRODUCTS
PROCEDE DE PRODUCTION DE DERIVES D'ACIDE 2-HALOGENODICOTINIQUE ET DE N-BUTYLESTER D'ACIDE 2-CHLORONICOTINIQUE COMME PRODUIT INTERMEDIAIRE

(30) Priorität: 31.07.1998 DE 19834565
(43) Veröffentlichungstag der Anmeldung: 30.05.2001
(73) Patentinhaber: BASF Aktiengesellschaft, 67056 Ludwigshafen (DE)
(72) Erfinder: GOLSCH, Dieter, D-67069 Ludwigshafen (DE); KEIL, Michael, D-67251 Freinsheim (DE); ISAK, Heinz, D-67459 Böhl-Iggelheim (DE); MAYER, Horst, D-67069 Ludwigshafen (DE)
(86) Internationale Anmeldenummer: PCT/EP1999/004967
(87) Internationale Veröffentlichungsnummer: WO 2000/007989

(56) Entgegenhaltungen:
- WO-A-99/37630
- DE-A- 3 840 954

## Beschreibung

Die vorliegende Erfindung betrifft ein Verfahren zur Herstellung von 2-Halogennikotinsäurederivaten der Formel I, 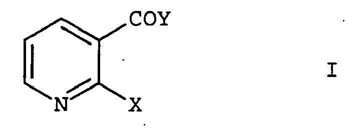 in der X die Bedeutung Brom oder Chlor hat und Y für Hydroxy, Brom oder Chlor steht.

Desweiteren wurde 2-Chlornikotinsäure-n-butylester als Zwischenprodukt gefunden.

2-Halogennikotinsäurederivate sind wichtige Zwischenprodukte zur Herstellung von beispielsweise pestiziden Wirkstoffen wie sie in den Patentschriften EP-A 53 011, EP-A 256 503 und EP-A 545 099 beschrieben sind.

Aus dem Stand der Technik sind bereits Verfahren zur Herstellung von 2-Halogennikotinsäureestern durch Ringschluß von 5-Aminopentadiensäureestern mit Halogenwasserstoff in polaren, protischen Lösungsmitteln wie Essigsäure oder Alkoholen bekannt (DE-A 38 40 954, EP-A 752 424, J. Org. Chem 41 (1976) S. 2066). In den bisher bekannten Verfahren werden ausschließlich die relativ polaren Pentadiensäure(m)ethylester eingesetzt. Durch Ringschluß entstehen somit 2-Halogennikotinsäure(m)ethylester, die selbst sehr polar sind und sich von den gleichfalls bei der Reaktion gebildeten polaren, teilweise teerartigen Nebenprodukten nur sehr schwer abtrennen lassen. Weiterhin fallen bei der Aufarbeitung wäßrige Waschbrühen an, die aufgrund der hohen Wasserlöslichkeit der Nikotinsäure(m)ethylester im Sauren große Mengen Wertprodukt enthalten. Zur Realisierung hoher Ausbeuten muß das Waschwasser daher zunächst mit Lauge teilweise neutralisiert werden, anschließend lassen sich die Nikotinsäure(m)ethylester durch aufwendige extraktive Verfahren rückgewinnen. Zur teilweisen Neutralisation der stark sauren Waschbrühen werden große Mengen an Lauge verbraucht. Zudem fallen bereits bei einem pH-Wert größer 0,5 teerartige Nebenprodukte aus, die eine Rückgewinnung des in Wasser gelösten Nikotinsäureesters erheblich erschweren.

Aufgabe der vorliegenden Erfindung war es daher ein wirtschaftliches, scale-up fähiges Verfahren zu finden, das eine einfache Aufarbeitung gewährleistet und dennoch hohe Ausbeuten liefert.

Demgemäß wurde das eingangs erwähnte Verfahren gefunden, daß dadurch gekennzeichnet ist, daß man
a) ein Pentadiensäureester der Formel II, 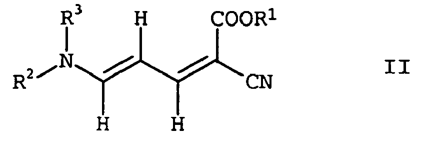 in der R¹ die Bedeutung n-Butyl besitzt und R² und R³ gleich oder verschieden sind und unabhängig voneinander C₁-C₄-Alkyl bzw. gemeinsam mit dem sie tragenden N-Atom einen fünf- bis siebengliedrigen heterocyclischen Ring bedeuten, mit Chlor- bzw. Bromwasserstoff umsetzt und
b) den so erhaltenen Nikotinsäureester I', 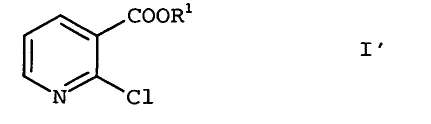 wobei R¹ für n-Butyl steht, im alkalischen zur Nikotinsäure I, wobei Y für Hydroxy steht, hydrolysiert und schließlich
c) gegebenenfalls die so erhaltene Nikotinsäure in das entsprechende Säurehalogenid I, wobei Y für Brom oder Chlor steht, überführt.

Die einzelnen Verfahrensschritte werden am Beispiel der Synthese der 2-Chlornikotinsäurederivate IA, IB und IC ausgehend von 2-Cyano-5-dimethylaminopentadiensäure-n-butylester näher erläutert (s. Schema 1). 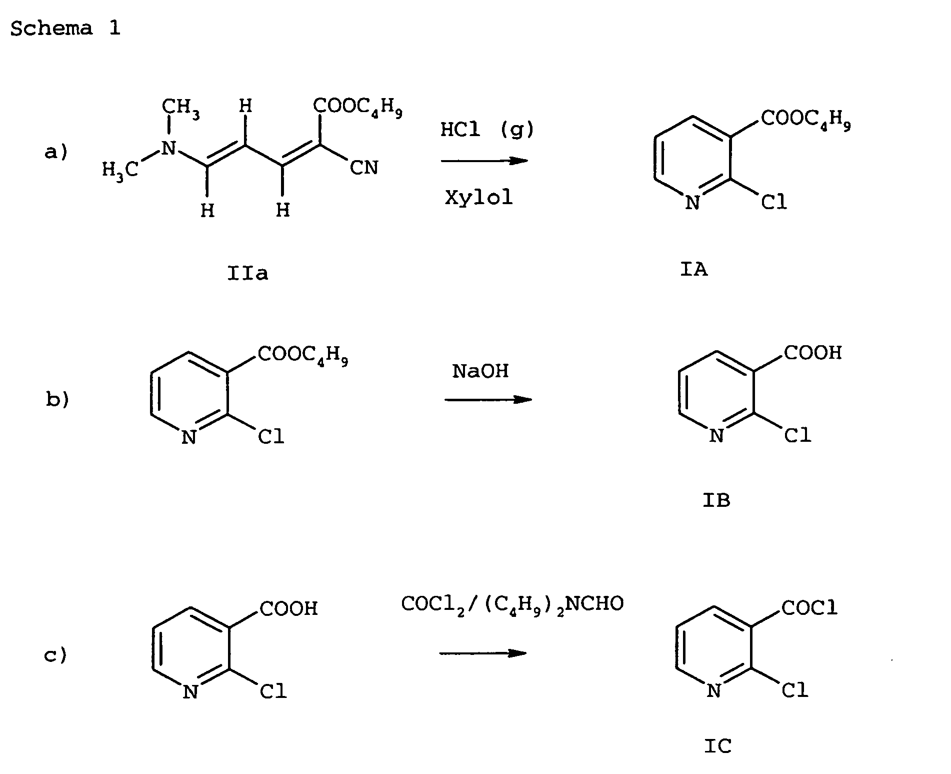
a) Zur Herstellung der 2-Halogennikotinsäureester I sind 5-Aminopentadiensäureester der Formel II geeignet, in der R¹ wie eingangs erwähnt n-Butyl bedeutet. Die Aminosubstituenten R² und R³ der Verbindungen II bedeuten jeweils vorzugsweise C₁-C₄-Alkyl und insbesondere jeweils Methyl.
   Als Lösungsmittel kommen polare Flüssigkeiten wie Essigsäure, Alkohole wie Methanol, Ethanol, n-Propanol, Isopropanol, n-Butanol oder Isobutanol in Frage. Bevorzugt werden unpolare Lösungsmittel wie Chlorbenzol und insbesondere bevorzugt Alkylbenzole wie Toluol oder Xylol eingesetzt. Insbesondere bevorzugt werden weiterhin Lösungsmittelgemische aus einem Alkylbenzol und beispielsweise Essigsäure verwendet. Hierbei wird das Alkylbenzol in Bezug auf die Essigsäure in der Regel in einem Gewichtsverhältnis von 10 : 1 bis 1 : 1 und vorzugsweise von 7 : 1 bis 3 : 1 eingesetzt.
   Die Umsetzung erfolgt üblicherweise bei Temperaturen von 40 bis 100°C und vorzugsweise bei 50 bis 75°C.
   Der Halogenwasserstoff wird in der Regel im 2 bis 10fachen und vorzugsweise im 3 bis 5fachen molaren Überschuß im Verlauf von 1 bis 5 und vorzugsweise 2 bis 4 Stunden eingeleitet. Mittels Bromwasserstoff gelangt man zu den 2-Bromnikotinsäureestern. Bevorzugt wird Chlorwasserstoff verwendet, womit 2-Chlornikotinsäureester zugänglich gemacht werden.
   Vorzugsweise wird der Halogenwasserstoff, insbesondere der Chlorwasserstoff unter 0,5 bis 6 bar Druck eingeleitet. Die Einleitungsphase des Chlorwasserstoffs kann dadurch auf unter eine halbe Stunde verkürzt werden. Bei der Fahrweise unter Druck betragen die Temperaturen während der Einleitphase vorzugsweise 10-45°C und während der ein- bis mehrstündigen Nachrührzeit 60-70°C.
   Die Aufarbeitung erfolgt im allgemeinen durch Waschen mit Wasser. Die erfindungsgemäßen höheren Nikotinsäureester liegen auch im stark Sauren nahezu ausschließlich in der organischen Phase vor. Auf eine teilweise Neutralisation des sauren Waschwassers (in der Regel liegt ein pH von 0 bis -1 vor) kann daher verzichtet werden. Damit ergeben sich mehrere Vorteile des erfindungsgemäßen Verfahrens. Zum einen können ca. zwei Äquivalente Lauge eingespart werden und weiterhin werden die teerartigen Ablagerungen vermieden, welche sich bei der teilweisen Neutralisation der sauren Waschbrühen auf pH ca. 2 ergeben. Diese Ablagerungen erschweren die Phasentrennung im Labormaßstab erheblich. Im technischen Maßstab können die einmal gebildeten teerartigen Ablagerungen nicht abgetrennt werden. Die Phasentrennung läßt sich hier nur realisieren, wenn bei einem pH kleiner 0,5 aufgearbeitet wird. Jedoch ist diese Verfahrensweise aufgrund des hohen Verlusts an 2-Halogennikotinsäure(m)ethylesters nicht wirtschaftlich.
   Die organische Phase enthält nahezu den gesamten 2-Halogennikotinsäureester, der beispielsweise destillativ aufgereinigt werden kann. Für die Herstellung der 2-Halogennikotinsäure kann die organische Phase auch direkt, ohne Abtrennung des Lösungsmittels eingesetzt werden.
b) Für die Hydrolyse der 2-Halogennikotinsäureester eignen sich beispielsweise wäßrige Lösungen von Natrium- oder Kaliumhydroxid, Natrium- oder Kaliumcarbonat. Vorzugsweise wird 5 bis 30%ige und insbesondere bevorzugt 10 bis 20%ige Natronlauge eingesetzt. Die Natronlauge kann zum einen vorgelegt werden.
   Hierzu wird der Nikotinsäureester in einem organischen Lösungsmittel gelöst (s. Schritt a)) oder vorzugsweise in reiner Form im Verlauf von bis zu 2 Stunden zudosiert.
   Bei der Hydrolyse wird vorzugsweise der Ester, ggf. mit einem Lösungsmittel verdünnt, vorgelegt und die Laugen zudosiert. Diese Fahrweise erlaubt mildere Reaktionsbedingungen (pH: 12 bis 13,5, Reaktionstemperatur: 40-60°C) und kürzere Reaktionszeiten von 0,5 bis 1 Stunde.
   Die Hydrolyse wird im allgemeinen bei 30 bis 80°C und vorzugsweise bei 40 bis 60°C durchgeführt. Der abgespaltene n-Butyl-Alkohol scheidet sich als separate Phase ab, was eine Rückgewinnung außerordentlich erleichtert und einen weiteren Vorteil dieser bevorzugten Ausführungsform des Verfahrens darstellt. In der Regel wird bis zu 5 Stunden, vorzugsweise bis zu 0,5 Stunden bei der obengenannten Temperatur nachgerührt.
   Die 2-Halogennikotinsäure wird in der Regel durch Ansäuern der wäßrigen Phase mit beispielsweise einer Mineralsäure wie Salzsäure oder Schwefelsäure ausgefällt. Zur besseren Kristallisation kann die Reaktionsmischung während des Fällvorgangs auf unter 25°C gekühlt werden.
   Zur Aufarbeitung wird der gebildete Niederschlag üblicherweise filtriert und getrocknet.
   Die Hydrolyse des 2-Chlornikotinsäure-n-butylesters zur 2-Chlornikotinsäure verläuft besonders glatt. Es wird ein gutsaugbares Produkt mit geringer Restfeuchte erhalten.
c) Die Überführung der 2-Halogennikotinsäure in das entsprechende Säurehalogenid kann mit den in der Literatur üblichen Halogenierungsmitteln geschehen. Zur Herstellung der 2-Halogennikotinsäurechloride eignen sich insbesondere Thionylchlorid und Phosgen. Im allgemeinen werden 1 bis 3 Äquivalente des Halogenierungsmittels bezogen auf die Säure eingesetzt.
   Als Lösungsmittel sind insbesondere aliphatische und aromatische Kohlenwasserstoffe geeignet.
   Die Reaktionstemperatur beträgt in der Regel 25 bis 150°C und vorzugsweise 70 bis 120°C.
   Vorzugsweise wird die Halogenierung in Gegenwart eines Katalysators durchgeführt. Als Katalysatoren sind beispielsweise Triphenylphosphin, Triphenylphosphinoxid und vorzugsweise Dialkylformamide wie N,N-Dimethylformamid, N,N-Diethylformamid, N,N-Di(n-butyl)formamid, N,N-Di(sec.-butyl)formamid geeignet. Insbesondere bevorzugt wird N,N-Di(n-butyl)formamid.

Die 5-Aminopentadiensäureester der Formel II' lassen sich vorzugsweise durch Knoevenagel-Kondensation von 3-Aminoacrolein III und Cyanessigsäureestern IV gewinnen (s. Schema 2). Grundsätzlich sind jedoch auch die beispielsweise in der EP-A 752 424 und in J. Org. Chem. 41 (1976) 2066 angegebenen Methoden zu deren Herstellung geeignet.

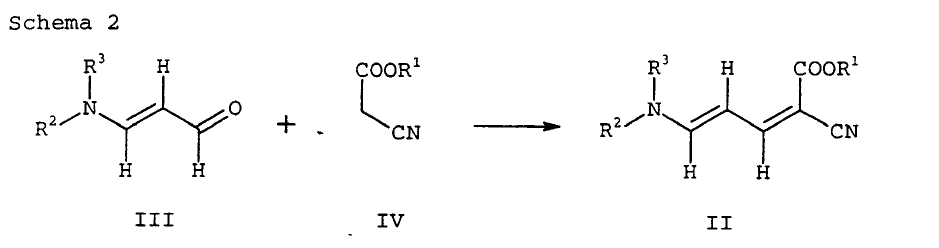

Das bei der Reaktion gemäß Schema 2 gebildete Wasser wird vorzugsweise mit Hilfe eines Wasserauskreisers kontinuierlich aus dem Reaktionsgemisch entfernt. Als Lösungsmittel, das gleichzeitig auch als Schleppmittel geeignet ist, kann vorzugsweise Toluol oder Xylol eingesetzt werden. Bei der Verwendung von Xylol als Schleppmittel kann bei Unterdruck gearbeitet werden, sodaß der Siedepunkt der Reaktionsmischung 110°C nicht übersteigt. Das Ende der Kondensation läßt sich anhand der Menge des gebildeten Wassers leicht ermitteln.

Im allgemeinen werden dem Reaktionsgemisch die in Knoevenagel-Kondensationen üblichen Katalysatoren zugesetzt. Besonders eignen sich hierfür sekundäre und inbesondere primäre Amine in Kombination mit einer Säure wie beispielsweise Essigsäure. Beispielsweise ist eine Katalysatormischung geeignet, die 1 bis 10 mol%, vorzugsweise 3-5% eines Monoalkylamins und 10 bis 50 mol%, vorzugsweise 15-20% Essigsäure enthält. Bevorzugt sind C₁-C₈-Monoalkylamine wie beispielsweise n-Butylamin oder n-Octylamin.

Am Ende der Reaktion wird die organische Phase abgetrennt. Hieraus läßt sich entweder der 5-Aminopentadiensäureester durch Abdestillieren des Lösemittels in Substanz gewinnen oder vorzugsweise kann die organische Phase auch direkt in die unter a) beschriebene Ringschlußreaktion eingesetzt werden (s. Beispiele).

Mit dem erfindungsgemäßen Verfahren sind 2-Chlornikotinsäureester der Formel I', 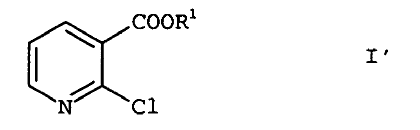 in der R¹ für n-Butyl steht, zugänglich, die sich beispielsweise hervorragend als Zwischenprodukte zur Herstellung von 2-Chlornikotinsäure eignen.

### Herstellungsbeispiele

### Vorprodukte

### 2-Cyano-5-dimethylaminopentadiensäure-n-butylester

104 g (1 mol) 3-Dimethylaminoacrolein, 0,575 1 (0,5 kg) Xylol, 3,9 g *n*-Octylamin (3 mol%) und 129 g Essigsäure (20 mol%) wurden vorgelegt und zum Sieden erhitzt. Hierzu wurden 152 g Cyanessigsäure-n-butylester (95%ig; 1,02 mol) innerhalb von 0,5 Stunden getropft. Währenddessen und während der anschließenden 2stündigen Nachrührzeit wurde das bei der Reaktion entstehende Wasser ausgekreist. Das Lösungsmittel wurde abdestilliert und die Titelverbindung in einer Rohausbeute von 97% gewonnen. Nach Umkristallisation aus Isopropanol wurden in 81% Ausbeute Kristalle mit einem Schmelzpunkt von 89°C erhalten.

### Nikotinsäurederivate

### Stufe a)

### Beispiel 1

### 2-Chlornikotinsäure-n-butylester

1 mol 2-Cyano-5-dimethylaminopentadiensäure-n-butylester wurden in 0,575 1 (0,5 kg) Xylol und 100 g Essigsäure vorgelegt. Bei 65°C wurden innerhalb von 3-4 Stunden 146 g (4 mol) Chlorwasserstoff eingeleitet. Eine Stunde wurde bei 65°C nachgerührt, anschließend wurde zweimal mit Wasser gewaschen. Dann wurde das Lösungsmittel bei einem Druck < 100 mbar abdestilliert. Nach Destillation bei einem Kp_{20mbar} von 156°C konnte das Wertprodukt in 96% Ausbeute erhalten werden.

### Beispiel 2

### 2-Chlornikotinsäure-n-butylester (Eintopf-Synthese ausgehend von 3-Dimethylaminoacrolein)

Vorgelegt wurden 110 g (1 mol) 3-Dimethylaminoacrolein (90 %ig), 3,9 g (3 mol%) n-Octylamin und 12 g (20 mol%) Essigsäure in 575 ml Xylol. Die Mischung wurde bei einem Druck von 400 mbar zum Sieden erhitzt und anschließend wurden innerhalb einer Stunde 148 g (1,05 mol) Cyanessigsäure-n-butylester zugetroft. Das bei der Reaktion entstehende Wasser wurde kontinuierlich ausgekreist. Als kein Wasser mehr überging, wurde auf 65°C abgekühlt und in die 65°C warme Lösung unter 2 bar Druck innerhalb von 10-30 Minuten 146 g (4 mol) Chlorwasserstoff bei 15-50°C eingeleitet. Anschließend wurde 3 Stunden bei 65°C nachgerührt. Die Reaktionslösung wurde mit Wasser gewaschen. Nach der Phasentrennung wurden 926 g organische Phase mit einem HPLC-Gehalt von 19,9% gewonnen, was einer Ausbeute von 86% an 2-Chlornikotinsäure-n-butylester entspricht. Zunächst wurde das Lösungsmittel abdestilliert und schließlich das Wertprodukt bei einem Kp_{20mbar} von 156°C in 80% Ausbeute überdestilliert.

### Beispiel 3

### 2-Cyano-5-dimethylaminopentadiensäure-2-ethylhexylester (Eintopf-Synthese ausgehend von 3-Dimethylaminoacrolein)

Die Synthese wurde analog Beispiel 2 ausgeführt und liefert in nahezu gleicher Ausbeute die Titelverbindung mit einem Kp_{0,9mbar} von 161°C.

### Beispiel 4 (Vergleich)

### 2-Chlornikotinsäureethylester (Eintopf-Synthese ausgehend von 3-Dimethylaminoacrolein)

Analog Beispiel 2 wurden ausgehend von 110 g (1 mol) 3-Dimethylaminoacrolein (90%ig) und 120 g (1,05 mol) Cyanessigsäureethylester und anschließendem Ringschluß unter Einleitung von 146 g (4 mol) Chlorwasserstoff 912 g organische Phase mit einem HPLC-Gehalt von 13,0% gewonnen, was einer Ausbeute von 67% an 2-Chlornikotinsäurebutylester entspricht. Aus der organischen Phase wurde zunächst das Lösungsmittel und schließlich das Wertprodukt bei einem Kp_{20mbar} von 143°C in einer Ausbeute von 65% überdestilliert.

### Beispiel 5 (Vergleich)

### 2-Chlornikotinsäureethylester (Eintopf-Synthese ausgehend von 3-Dimethylaminoacrolein)

Es wurde wie in Beispiel 4 verfahren, jedoch wurde die nach dem Ringschluß erhaltene wäßrige Phase, die zunächst einen pH von -0,7 aufwies, mit 25%iger NaOH auf pH 0.5 gebracht. Zwischen wäßriger und organischer Phase bildete sich eine Teerschicht. Die Phasentrennung gelang erst nach Zusatz weiterer Mengen Xylol. Es wurden 1540 g organische Phase erhalten mit einem HPLC-Gehalt von 9,3%, was einer Ausbeute von 78% an 2-Chlornikotinsäurebutylester entspricht. Aus der organischen Phase wurde zunächst das Lösungsmittel und schließlich das Wertprodukt bei einem Kp_{20mbar} von 143°C in 66% Ausbeute überdestilliert.

### Stufe b)

### Beispiel 6

### 2-Chlornikotinsäure

### i) Vorlage der Lauge

96 g NaOH (1,2 mol) wurden als 50%ige wäßrige Lösung vorgelegt und auf 60°C erwärmt. Innerhalb einer halben Stunde wurden 213 g (1 mol) 2-Chlornikotinsäure-n-butylester zugetropft. Die Reak- tionsmischung wurde noch eine halbe Stunde bei dieser Temperatur nachgerührt. Die organische Phase wurde mit 50 ml Wasser gewaschen und die wässrigen Phasen vereinigt. Mit 20 %iger HCl bei 40-15°C wurde ein pH von 1,2 eingestellt, das ausgefallene Wertprodukt abfiltriert, mit 0,5 1 kaltem Wasser gewaschen und getrocknet. Die Ausbeute der Titelverbindung betrug 99%.

### ii) Vorlage des Esters

250 g 2-Chlornikotinsäure-n-butylester (95 Gew.-% entspricht 1,1 mol) wurden in 142,4 g Wasser vorgelegt und auf 45°C erwärmt. Innerhalb von 50 Minuten wurden 213 g 25 %ige NaOH (1,33 mol) bei dieser Temperatur zudosiert und anschließend noch ca. 30 Minuten bei dieser Temperatur nachgerührt. Die Aufarbeitung erfolgte analog Beispiel 6i). Die Ausbeute der Titelverbindung betrug 96,4 %.

### Stufe c)

### Beispiel 7

### 2-Chlornikotinsäurechlorid

Bei Raumtemperatur wurden 800 g (5,08 mol) 2-Chlornikotinsäure 1800 ml Xylol und 8 g (1 mol%) Di(n-butyl)formamid vorgelegt und auf 100°C erwärmt. Bei dieser Temperatur wurden innerhalb von 3 Stunden 1005 g (10,15 mol) Phosgen eingeleitet. Nach Abdestillieren des Lösungsmittels wurde das Säurechlorid bei 73-85°C und einem Druck von 1-2 mbar destilliert (Ausbeute 94,5%).

## Patentansprüche

1. Verfahren zur Herstellung von 2-Halogennikotinsäurederivaten der Formel I, 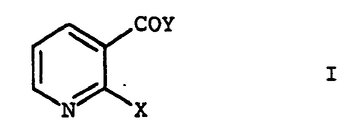 in der X die Bedeutung Brom oder Chlor hat und Y für Hydroxy, Brom oder Chlor steht, **dadurch gekennzeichnet, daß** man
a) ein Pentadiensäureester der Formel II, 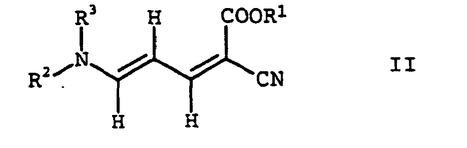 in der R¹ die Bedeutung n-Butyl besitzt und R² und R³ gleich - oder verschieden sind und unabhängig voneinander C₁-C₄-Alkyl bzw. gemeinsam mit dem sie tragenden N-Atom einen fünf- bis siebengliedrigen heterocyclischen Ring bedeuten, mit Chlor- bzw. Bromwasserstoff umsetzt und
b) den so erhaltenen Nikotinsäureester I', 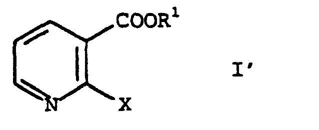 wobei R¹ für n-Butyl steht, im alkalischen zur Nikotinsäure I, wobei Y für Hydroxy steht, hydrolysiert und schließlich
c) gegebenenfalls die so erhaltene Nikotinsäure in das entsprechende Säurehalogenid I, wobei Y für Brom oder Chlor steht, überführt.

2. Verfahren gemäß Anspruch 1 zur Herstellung von 2-Chlornikotinsäure, **dadurch gekennzeichnet, daß** ein Pentadiensäureester der Formel II gemäß Anspruch 1 mit Chlorwasserstoff bei einem Druck von 0,5 bis 6 bar umgesetzt wird und anschließend hydrolysiert wird.

3. Verfahren gemäß Anspruch 1, **dadurch gekennzeichnet, daß** bei der Ringschlußreaktion a) als Lösungsmittel ein aromatischer Kohlenwasserstoff verwendet wird.

4. Verfahren gemäß Anspruch 1, **dadurch gekennzeichnet, daß** im Schritt c) die Herstellung des Säurehalogenids in Gegenwart katalytischer Mengen Di(n-butyl)formamid erfolgt.

5. Verfahren gemäß Anspruch 2, **dadurch gekennzeichnet, daß** der Pentadiensäureester der Formel II gemäß Anspruch 1 durch Kondensation eines 3-Aminoacroleins der Formel III, 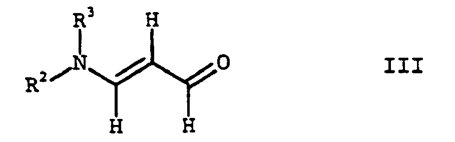 in der R² und R³ die in Anspruch 1 genannte Bedeutung besitzen, und eines Cyanessigsäureesters der Formel IV, 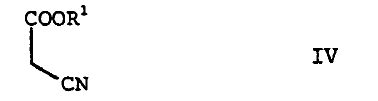 in der R¹ die in Anspruch 1 genannte Bedeutung besitzt, hergestellt wird.

6. Verfahren gemäß Anspruch 5, **dadurch gekennzeichnet, daß** die Kondensation des 3-Aminoacroleins III und des Cyanessigsäureesters IV zum Pentadiensäureester II und der Ringschluß von II zum Nikotinsäureester I' gemäß Anspruch 1 ohne Zwischenisolierung des Pentadiensäureesters II erfolgt.

7. 2-Chlornikotinsäure-n-butylester.

## Claims

1. A process for the preparation of 2-halonicotinic acid derivatives of the formula I 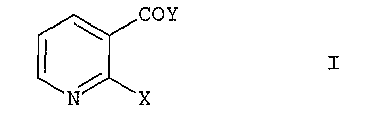 in which X denotes bromine or chlorine and Y is hydroxyl, bromine or chlorine, which comprises
a) reacting a pentadienoic ester of the formula II 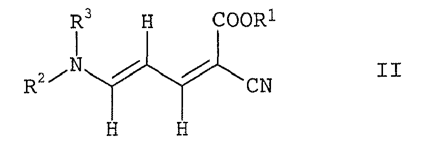 in which R¹ denotes n-butyl and R² and R³ are identical or different and independently of one another are C₁-C₄-alkyl or together with the N atom to which they are attached are a 5-to 7-membered heterocyclic ring with hydrogen chloride or hydrogen bromide, and
b) hydrolyzing the resulting nicotinic ester I' 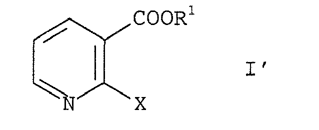 in which R¹ is n-butyl, in alkaline medium to give nicotinic acid I in which Y is hydroxyl, and finally
c) if appropriate, converting the resulting nicotinic acid into the corresponding acid halide I in which Y is bromine or chlorine.

2. The process according to claim 1 for the preparation of 2-chloronicotinic acid, which comprises reacting a pentadienoic ester of the formula II according to claim 1 with hydrogen chloride at a pressure of 0.5 to 6 bar, with subsequent hydrolysis.

3. The process according to claim 1, wherein an aromatic hydrocarbon is used as solvent in the cyclization reaction a).

4. The process according to claim 1, wherein, in step c), the acid halide is prepared in the presence of catalytic amounts of di(n-butyl)formamide.

5. The process according to claim 2, wherein the pentadienoic ester of the formula II according to claim 1 is prepared by subjecting a 3-aminoacrolein of the formula III 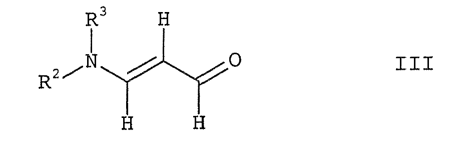 in which R² and R³ have the meanings given in claim 1, and a cyanoacetic ester of the formula IV 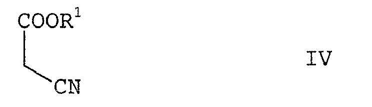 in which R¹ is as defined in claim 1, to a condensation reaction.

6. The process according to claim 5, wherein the condensation of the 3-aminoacrolein III and of the cyanoacetic ester IV to the pentadienoic ester II and cyclization of II to the nicotinic ester I' according to claim 1 is carried out without intermediate isolation of the pentadienoic ester II.

7. n-Butyl 2-chloronicotinate.

## Revendications

1. Procédé de préparation de dérivés d'acide 2-halogénonicotinique de formule I, 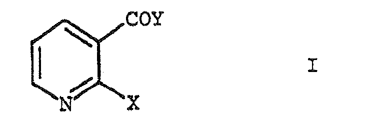 dans laquelle X signifie brome ou chlore, et Y représente un groupe hydroxy, du brome ou du chlore, **caractérisé en ce que**,
a) un ester d'acide pentadiénique de formule II, 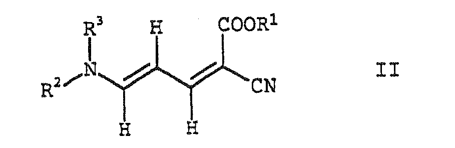 dans laquelle R¹ signifie n-butyle et R² et R³ sont identiques ou différents et signifient, indépendamment l'un de l'autre, alkyle en C₁-C₄ ou, en commun avec l'atome de N qui les porte, un cycle hétérocyclique pentagonal à heptagonal, est amené à réagir avec du chlorure d'hydrogène ou du bromure d'hydrogène et
b) l'ester d'acide nicotinique I' ainsi obtenu, 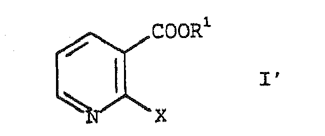 R¹ représentant du n-butyle, est hydrolysé de manière alcaline pour donner l'acide nicotinique I, Y représentant un groupe hydroxy, et ensuite
c) l'acide nicotinique ainsi obtenu est éventuellement converti en l'halogénure d'acide I correspondant, Y représentant du brome ou du chlore.

2. Procédé selon la revendication 1 de préparation d'acide 2-chloronicotinique, **caractérisé en ce qu'**un ester d'acide pentadiénique de formule II selon la revendication 1 est amené à réagir avec du chlorure d'hydrogène à une pression de 0,5 à 6 bars et est ensuite hydrolysé.

3. Procédé selon la revendication 1, **caractérisé en ce qu'**un hydrocarbure aromatique est utilisé en tant que solvant dans la réaction de fermeture de cycle a) .

4. Procédé selon la revendication 1, **caractérisé en ce que** la préparation de l'halogénure d'acide a lieu dans l'étape c) en présence de quantités catalytiques de di(n-butyl)formamide.

5. Procédé selon la revendication 2, **caractérisé en ce que** l'ester d'acide pentadiénique de formule II selon la revendication 1 est préparé par condensation d'une 3-aminoacroléine de formule III, 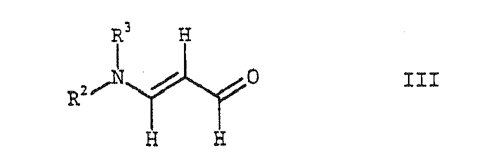 dans laquelle R² et R³ ont la signification donnée dans la revendication 1, et d'un ester d'acide cyanoacétique de formule IV, 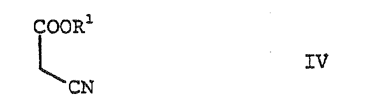 dans laquelle R¹ a la signification donnée dans la revendication 1.

6. Procédé selon la revendication 5, **caractérisé en ce que** la condensation de la 3-aminoacroléine III et de l'ester d'acide cyanoacétique IV pour donner l'ester d'acide pentadiénique II et la fermeture de cycle de II pour donner l'ester d'acide nicotinique I' selon la revendication 1 a lieu sans isolation intermédiaire de l'ester d'acide pentadiénique II.

7. Ester n-butylique d'acide 2-chloronicotinique.
